# EUROPEAN PATENT SPECIFICATION

(11) **EP 2 175 918 B1**
(45) Date of publication and mention of the grant of the patent: **13.01.2016**
(21) Application number: 08781486.9
(22) Date of filing: 08.07.2008
(51) Int. Cl.: A61M 11/00, A61M 15/00, B28B 1/24

(54) **MANUFACTURE OF COMPONENTS FOR MEDICINAL DISPENSERS**
HERSTELLUNG VON BESTANDTEILEN MEDIZINISCHER SPENDER
FABRICATION DE COMPOSANTS POUR DISTRIBUTEURS MÉDICINAUX

(30) Priority: 18.07.2007 GB 0713876
(43) Date of publication of application: 21.04.2010
(73) Proprietor: 3M Innovative Properties Company, Saint Paul, MN 55133-3427 (US)
(72) Inventor: MCGLASSON, Stuart A., Bracknell Berkshire RG12 8HT (GB); HODSON, Peter D., Bracknell Berkshire RG128HT (GB); ALEANDRI-HACHGENEI, Lorraine E., 41453 Neuss (DE)
(74) Representative: Lanoe, Benjamin
(86) International application number: PCT/US2008/069400
(87) International publication number: WO 2009/012078

(56) References cited:
- GB-A- 2 328 932
- GB-A- 2 417 479
- GB-A- 2 430 188
- US-A- 6 119 853
- US-A- 6 156 246
- US-A1- 2002 190 085
- US-B1- 6 240 918
- US-B1- 6 978 915
- US-B2- 7 234 460
- GERMAN R M ET AL: "KEY ISSUES IN POWDER INJECTION MOLDING", AMERICAN CERAMIC SOCIETY BULLETIN, AMERICAN CERAMIC SOCIETY. COLUMBUS, US, vol. 70, no. 8, 1 August 1991 (1991-08-01) , pages 1294-1295,1297, XP000223773, ISSN: 0002-7812

## Description

### Technical Field

The present invention relates generally to the manufacture and provision of components for medicinal pressurized metered dose dispensers (e.g. pressurized metered dose inhalers) for dispensing pharmaceutical aerosol formulations, more particularly to manufacture and provision of valve components (e.g. valve stems and valve bodies) for use in pressurized metered dose dispensing valves as well as aerosol containers for use in medicinal pressurized metered dose dispensers (e.g. pressurized metered dose inhalers).

### Background

The use of aerosols to administer medicament has been known for several decades. Such aerosol formulations generally comprise medicament, one or more propellants, (e.g. chlorofluorocarbons and more recently hydrogen-containing fluorocarbons, such as HFA 134a (CF₃CH₂F) and HFA 227 (CF₃CHFCF₃)) and, if desired, other excipients, such as a surfactant and/or a solvent, such as ethanol.

Pharmaceutical aerosols for inhalation, nasal, or sublingual administration generally comprise a container or vial of the aerosol formulation equipped with a metered dose dispensing valve to provide a dispensing canister. Although there are many different designs of metered dose valves, most comprise a metering chamber defined in part by a valve body and a valve stem that slides through a diaphragm into the metering chamber. When the valve is in its non-dispensing position, the diaphragm maintains a closed seal around the valve stem. The valve stem typically includes an opening (typically a side port) in communication with a discharge passageway inside the valve stem. When such a valve is actuated, the valve stem typically moves inwardly, so that the side port of the valve stem passes the diaphragm and enters into the metering chamber, allowing the contents of the metering chamber to pass through the side port and passageway, and to exit through the stem outlet.

US-B-6,156,246 discloses injection moulding of inorganic materials and more particularly low pressure injection moulding of particulate zirconia, its composites, and other particulate ceramics for manufacture of low cost, high precision, and complex shaped parts.

Amer. Ceram. Soc. Bull. 70 (1991) pp1294 to 1302 (German et al) relates to key issues in powder injection moulding.

GB-A-2,417,479 discloses improvements in metering valves for pressurized dispensing containers.

Other metered dose dispensing valves such as those described in patent applications WO 2004/022142 and WO 2004/022143 form a transient metering chamber upon actuation. For example WO 2004/022142 describes *inter alia,* a metered dose valve comprising a valve body having an internal chamber with a valve stem positioned therein which has a body portion that is generally triangular through to diamond shaped in its vertical cross-section and a stem portion in sealing engagement with a diaphragm seal. Upon initial inward movement of the valve stem of such a valve, the inwardly facing surface of the valve stem body portion forms a face seal with a metering gasket provided on the valve body thus forming a transient metering chamber between *inter alia* the outwardly facing surface of the valve stem body portion and a portion of the valve body. Upon further movement of the valve stem inwardly, an opening to a discharge passageway provided in the valve stem passes the diaphragm and the contents trapped within the transient metering chamber pass through the discharge passageway of the valve stem, exiting the stem outlet.

It will be appreciated that both the valve of a pressurized metered dose inhaler (pMDI) and also the pharmaceutical aerosol formulation each play an important part in obtaining optimum pharmaceutical performance from the product. In particular, pMDI valves represent a uniquely challenging application of metering valves, and generally need to meet many exacting requirements. For example the valve must be capable of adequately sealing pharmaceutical formulations based on pressurized, liquefied propellant systems, while minimizing any transmission of propellant out of the system and moisture into the system. Also the valve must be small and desirably inexpensive and must operate at suitably low actuation forces e.g. through reliable, smooth and easy movement of the valve stem. And of course upon actuation/operation of the valve stem, the valve must adequately sample and accurately meter the medicinal aerosol formulation.

The use of metal rather than plastic for valve stems and/or other internal valve components is frequently beneficial for minimizing distortion or material failure, e.g. of the valve stem upon use, as well as for avoiding many leachables from the plastic materials. Metal valve stems are for example conventionally constructed by deep drawing or machining. Some shapes and configurations of valve stems may not be optimally manufactured using deep drawing, however. Additionally the thin-walled nature of deep drawn valve stems can leave large internal voids that can present problems such as drug deposition that can lead to complete occlusion of the valve stem. Machined metal valve components such as valve stems, e.g. formed using forging, turning and/or drilling, are relatively expensive. Machining is also disadvantageous in that the surface finish of the machined valve component can be quite rough, e.g. through the presence of machining marks. While extensive polishing can be used to improve the finish of external surfaces of such a component, unfavorably rough surfaces which cannot be polished, for example internal walls of passages and/or openings thereto in the valve stem, can provide seeding surfaces for drug deposition/accumulation, which in the case of a valve stem can lead to occlusion of its internal passages and/or openings.

Also the container of a pMDI is important in obtaining optimum pharmaceutical performance from a product. For example, the container needs to contain the pharmaceutical aerosol formulation and be inert. Conventionally containers have been made of metal, plastic or glass, where metal containers are generally favored in order to avoid leachables from plastic material and to avoid any potential of breakage e.g. associated with glass containers. Internal walls of metal containers, such as deep drawn, aluminum or aluminum alloy containers, can disadvantageously provide seeding surfaces for drug deposition/accumulation.

### Summary

Surprisingly, it has been found that ceramic powder injection molding (which can also be called ceramic injection molding) is particularly useful for the manufacture and provision of components for medicinal pressurized metered dose dispensers (e.g. pMDIs) for dispensing pharmaceutical aerosol formulations, in particular valve components (e.g. valve stems and valve bodies) for use in pressurized metered dose dispensing valves as well as containers. The determined suitability of ceramic powder injection molding (referred to in the following as CPIM) is particularly surprising because in CPIM processes individual particles of a ceramic and/or a ceramic-precursor material are sintered to yield a ceramic component and although such processes would be expected to yield ceramic components having an unfavorable pebble-like surface finish and allowing for transmission of pressurized, liquefied propellant into and/or through said components, it has been found that CPIM can be used to provide components having highly desirable surface finishes, even without polishing, and desirable resistance to transmission of pressurized, liquefied propellant.

Accordingly one aspect of the present invention is the use of CPIM for the manufacture of a ceramic component of a medicinal pressurized metered dose dispenser.

A further aspect of the present invention is a method of manufacturing a ceramic component of a medicinal pressurized metered dose dispenser, said method comprising the steps of
a) providing a mold for the component,
b) injecting into the mold a feedstock of ceramic and/or ceramic-precursor particles in a binder to provide a green part,
c) removing binder to provide a brown part, and
d) sintering the brown part; wherein the ceramic particles and/or ceramic precursor particles are selected such that the manufactured component is made of a ceramic nitride or a ceramic nitride-containing composite.

In particular the component is a component that in its use in a medicinal pressurized metered dose dispenser (e.g. a pMDI) comes into contact with pharmaceutical aerosol formulation and/or pressurized, liquefied propellant, as applicable, during storage or dispensing.

Favorably the component may be a container or a valve component (e.g. a component of a metered dose dispensing valve).

The valve component may be a valve stem or a valve body, such as a valve body of which at least a portion thereof in part defines a (non-transitory or transitory) metering chamber (referred to in the following as primary valve bodies) or other types of valve bodies (referred to in the following as secondary valve bodies) which can define in part a pre-metering region/chamber and/or a spring cage and/or a bottle emptier. In addition the valve component may be any other type of valve component that in its use in a pressurized metered dose dispenser comes into contact with pharmaceutical aerosol formulation and/or pressurized, liquefied propellant.

CPIM is also advantageous in that it allows for the manufacture of components having various shapes (e.g. valve components of various complex shapes and configurations) to desirably tight tolerances. CPIM is hence particularly useful for the manufacture of primary valve bodies and valve stems, in particular valve stems. In regard to valve stems, CPIM is further advantageous in that it allows for the provision of internal passages and/or openings (e.g. side ports) thereto having desirable structural form and surface finish without a need for post-machining or finishing.

Another aspect of the present invention is a component of a medicinal pressurized metered dose dispenser that is made of a ceramic nitride or a ceramic nitride-containing composite.

A further aspect of the present invention is a component of a medicinal pressurized metered dose dispenser that is made of a ceramic selected from the group consisting of silicon nitride, silicon nitride-containing composite, aluminum nitride, aluminum nitridecontaining composite, silicon oxynitride, silicon oxynitride-containing composite, aluminum oxynitride, aluminum oxynitride-containing composite.

An additional aspect of the present invention is a medicinal pressurized metered dose dispenser e.g. a pressurized metered dose inhaler, comprising a component described herein. In further aspects, the present invention provides a metered dose dispensing valve comprising a valve component described herein and a pressurized metered dose dispenser, e.g. a pressurized metered dose inhaler, comprising such a metered dose dispensing valve.

The dependent claims define further favorable embodiments.

The invention, its embodiments and further advantages will be described in the following with reference to the following drawings.

### Brief Description of the Drawings

Figure 1 shows a cross-section through a metered dose dispensing valve.
Figure 2 shows a cross-section through an alternative metered dose dispensing valve.
Figure 3 shows a partial cross-section through a further metered dose dispensing valve.
Figure 4 shows a cross-section through a pressurized metered dose inhaler including the metered dose dispensing valve shown in Figure 3.

### Detailed Description

It is to be understood that the present invention covers all combinations of particular and preferred aspects of the invention described herein.

CPIM (ceramic powder injection molding) is generally understood to include processes including injecting particles of ceramic and/or ceramic-precursor material in a binder into a mold cavity and further processing allowing for removal of binder and sintering of particles to provide a ceramic component. It is understood that CPIM does not include compression molding or isostatic pressing processes wherein a slab of material is squeezed by mold halves. It is generally understood that a ceramic is any inorganic, non-metallic material that is crystalline or partly crystalline (preferably to the most part (greater than 50%) crystalline, more preferably at least 75% crystalline, most preferably at least 90% crystalline). Ceramic particles are particles made of inorganic, non-metallic material that are crystalline or partly crystalline. Ceramic-precursor particles may be made of any type of non-ceramic material (e.g. a glass (non-crystalline) material or metallic material or other types of suitable ceramic-precursor materials) which after processing via CPIM provides a ceramic material.

It has been found the CPIM is particularly useful for the manufacture of components for use in medicinal pressurized metered dose dispensers, such as pMDIs.

This holds particularly true for components which in their use in such dispensers come into contact or are in contact with pharmaceutical aerosol formulations and/or pressurized, liquefied propellant and/or have complex geometries. This holds especially true for containers as well as valve components (e.g. valve stems, valve bodies, such as primary valve bodies or secondary valve bodies) for metered dose dispensing valves for use in such dispensers, like pMDIs.

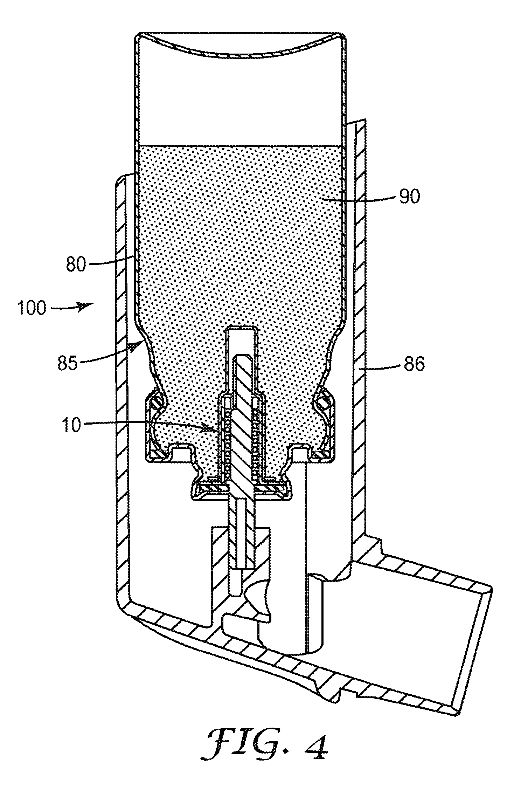

As mentioned above pharmaceutical aerosol formulations generally comprise medicament, one or more propellants, (e.g. chlorofluorocarbons and more recently hydrogen-containing fluorocarbons, such as HFA 134a (CF₃CH₂F) and HFA 227 (CF₃CHFCF₃)) and, if desired, other excipients, such as a surfactant and/or a solvent, such as ethanol. Figure 4 shows an exemplary pMDI (100) comprising a container (80) equipped with a metered dose dispensing valve (10) to provide a dispensing canister (85) which is generally located in an adaptor or housing (86). It is appreciated that a container of a medicinal pressurized metered dose dispenser contains pharmaceutical aerosol formulation (labeled 90 in Figure 4) wherein generally the propellant is pressurized enough to cause it to be liquefied, and thus during storage and dispensing the container is in contact with pharmaceutical aerosol formulation, more particularly and typically pressurized, liquefied propellant containing medicament and, if desired, other excipients. Containers of medicinal pressurized metered dose dispensers (e.g. pMDIs) are favorably manufactured via CPIM. Through the use of CPIM, such containers can be effectively and cost-efficiently manufactured, while allowing the provision of desirably inert, strong, light and clean (e.g. with an absence of leachables) containers. Due to the provision of highly desirable surface finishes such containers can be favorably used directly, e.g. without a need for further surface processing, such as applying a coating on an internal surface (such coatings typically being used to reduce deposition of particulate matter and/or to reduce interaction between the internal surface of the container and the contents of the container). Containers described herein are thus advantageously substantially free or free of a coating on an internal surface thereof. Containers described herein are advantageous for containing a pharmaceutical aerosol formulation comprising a particulate medicament (e.g. suspension aerosol formulations comprising (more particularly consisting essentially of, even more particularly, consisting of) a particulate medicament and a propellant selected from HFA 134a and/or HFA 227). Further as mentioned above, such containers are desirably strong, and thus can be used without a need to provide further structural strengthening, for example as described in WO 02/72449 disclosing a canister where its walls are formed of a laminate including a metal laminate layer having a minimum thickness of 0.1 mm. Walls of containers described herein are favorably free of a metal laminate layer having a thickness of 0.1 mm or greater, more favorably walls of containers described herein are free of a metal laminate layer. Although typically not necessary for structural reasons, if desired (e.g. for aesthetic reasons), an outer surface of such containers (e.g. dark or off-white containers) may be provided with a coating, for example a metallic or a pigmented coating. Generally such coatings, typically being decorative, are thin, e.g. at most 50 microns, typically about 0.2 to about 30 microns.

Metered dose dispensing valves for use in medicinal pressurized metered dose dispensers, such as pMDIs, typically comprise a valve stem co-axially slidable within a valve body (a primary valve body), an outer seal (e.g. diaphragm seal) and an inner seal (e.g. metering gasket). The outer and inner seals may be provided at the outer and inner ends of the valve body and the valve stem positioned in sliding sealing engagement with the seals so that a metering chamber is defined between the valve stem, valve body and seals. Alternatively the outer seal may be provided at the outer end of the valve body, the valve stem positioned in sliding sealing engagement with the outer seal, while the valve body, valve stem and inner seal are configured and positioned such that upon actuation of the valve, e.g. movement of the valve stem, the inner seal is operative to form a transient, fluid-tight seal between the valve stem and the valve body. In such valves a metering chamber, e.g. a transitory metering chamber, is favorably formed upon actuation. Metered dose dispensing valves generally comprise secondary valve bodies and depending on the particular design of the valve, such a secondary valve body can, for example, define a pre-metering region/chamber, a spring cage and/or a bottle emptier. Components of metered dose dispensing valves, in particular valve stems, primary valve bodies and/or secondary valve bodies of such valves are favorably manufactured via CPIM. Through the use of CPIM, valve components, even complex-shaped valve components, can be effectively and cost-efficiently manufactured, while allowing the provision of desirably inert, clean (e.g. with an absence of leachables), strong and wear-resistance valve components. Duc to the provision of highly desirable surface finishes such valve components can be favorably used directly, e.g. without a need for further surface processing, such as polishing or applying a coating (the purposes of which are described above) on their surface(s) that in normal use will come into contact with a pharmaceutical aerosol formulation and/or pressurized liquefied propellant. Valve components described herein arc thus advantageously substantially free or free of a coating on their surface(s) that in their routine use in a medicinal pressurized metered dose dispenser (e.g. a pMDI) come(s) into contact with pharmaceutical aerosol formulation and/or pressurized, liquefied propellant. Valve components described herein arc advantageous for use in a medicinal pressurized metered dose dispenser (e.g. a pMDI) containing a pharmaceutical aerosol formulation comprising a particulate medicament (e.g. suspension aerosol formulations comprising (more particularly consisting essentially of, even more particularly, consisting of) a particulate medicament and a propellant selected from HFA 134a and/or HFA 227). If desired (e.g. for aesthetic reasons), other surfaces (e.g. surfaces visible to the dispenser's user) of valve components (e.g. outer surface(s) of the external portion of the valve stem) may be provided with a coating, for example a metallic or a pigmented coating. As mentioned above, generally such coatings, typically being decorative, are thin, e.g. at most 50 microns, typically about 0.2 to about 30 microns.

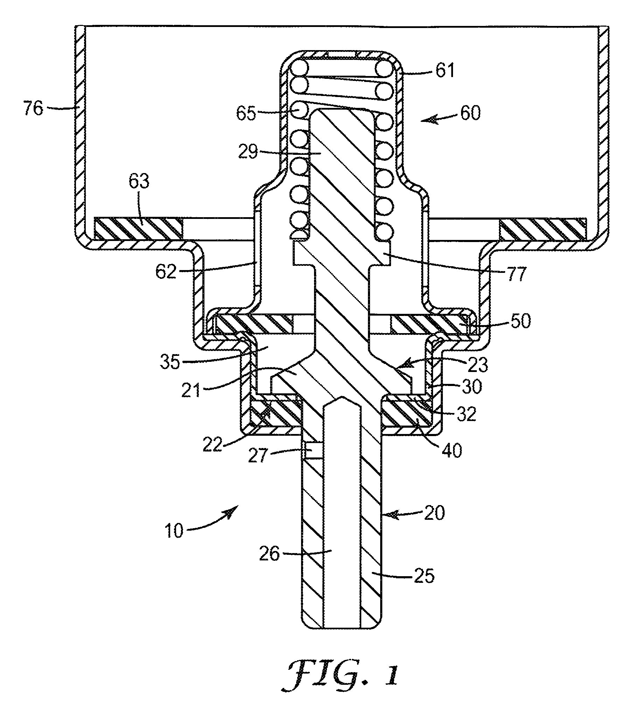

Figures 1-3 illustrate examples of metered dose dispensing valves which favorably include valve components manufactured via CPIM.

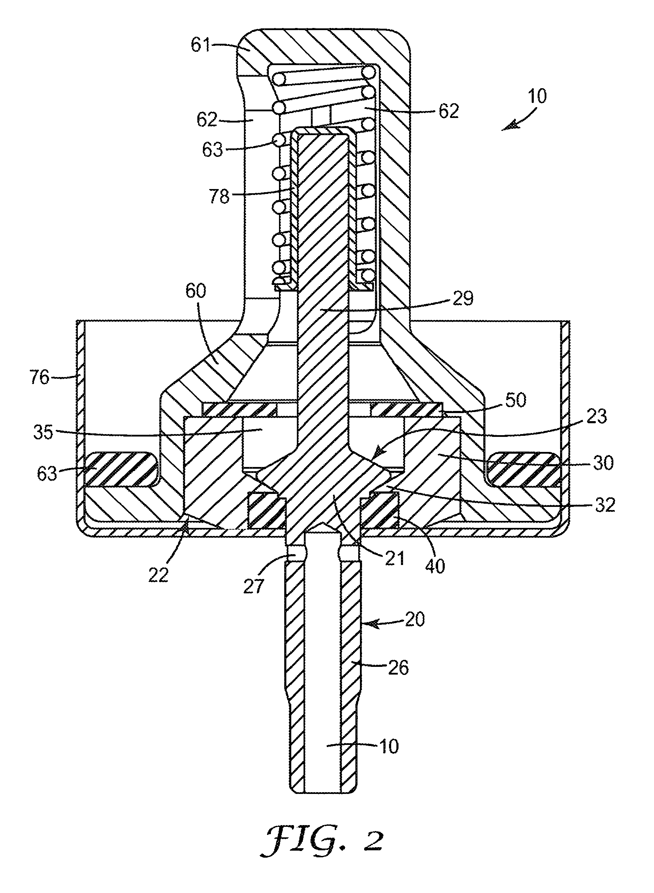

Figures 1 and 2 illustrate two embodiments of a metered dose dispensing valve (10) of the general type described in WO 2004/022142. In use, such valves are crimped onto an aerosol container (not shown) using a ferrule (76) where a gasket seal (63) is located between the valve ferrule and the opening of the aerosol container to ensure a gas-tight scal between the valve and the aerosol container. Formulation within the aerosol container will surround and contact inner components of the valve. Referring to Figures 1 and 2, in each exemplary embodiment, the valve comprises a valve stem (20) that generally defines a longitudinal axis and comprises a body portion (21) and a stem portion (25) including a discharge passageway (26), and a valve body (30), where an internal chamber (35) is defined at least in part by at least a portion of the inner surface of the valve body. The body portion (21) of the valve stem is generally positioned within a portion of the internal chamber (35). As can be recognized from Figures 1 and 2, the body portion of the valve may be generally triangular or diamond-shaped in its cross section. Moreover in such valves, the body portion of the valve stem favorably comprises a metering surface (22) near the stem portion (25) of the valve stem, wherein the longitudinal axis and a plane tangential to at least a portion of the metering surface define an angle from about 2° to about 90°. The embodiment shown in Figure 1 has an angle of about 90°, while the embodiment shown in Figure 2 has an angle of about 55°. Also as can be appreciated from Figures 1 and 2, the valve body (30) comprises a metering portion (32) the surface of which is configured to substantially conform to the metering surface of the valve stem. Each valve includes a diaphragm seal (40) having walls that define an aperture in slidable, sealing engagement with the stem portion (25) of the valve stem, and a metering gasket (50). The metering gasket (50) is configured and positioned such that upon actuation of the valve (e.g. movement of the valve stem inwardly) a transient, substantially fluid-tight face seal can be formed between the metering gasket and the valve stem (20), in particular between the body portion (21) of the valve stem, more particular a sealing surface (23) of the body portion. As will be appreciated by the skilled reader, upon movement of the valve stem inwardly a (transitory) metering chamber (not visible) is formed between the metering surface (22) of the valve stem and the metering portion (32) of the valve body and upon formation of the described face seal, aerosol formulation in the thus-formed metering chamber is isolated from the aerosol container. Upon further movement of the valve stem inwardly, an opening (27) into the discharge passageway (26) of the valve stem passes the diaphragm seal and the contents of the metering chamber pass through the discharge passageway of the valve stem, exiting the stem outlet. As can be appreciated from the embodiments shown in Figures 1 and 2, in such valves the sealing surface (23) of the body portion (21) of valve stem (20) is desirably generally conical or conical. Moreover it is favorable that the longitudinal axis and a plane tangential to at least a portion of the sealing surface define an angle from about 30° to about 80°. Referring to Figures 1 and 2, in cach exemplary embodiment, the valve typically comprises a second valve body (60) defining a spring cage (61) for holding a compression spring (65). One end of the compression spring abuts the inner, upper wall of the spring cage and the other end abuts either a flange (77) on an upper stem portion (29) of the valve stem (sec Figure 1) or a separate flange component (78) mounted onto the upper stem portion of the valve stem (see Figure 2). One or more inlets (62) typically traversing the spring cage provide open and substantially unrestricted fluid communication between the interior chamber (35) and the aerosol container (not shown). The valve stem (20), primary valve body (30) and/or secondary valve body (60) of such valves are favorably manufactured via CPIM.

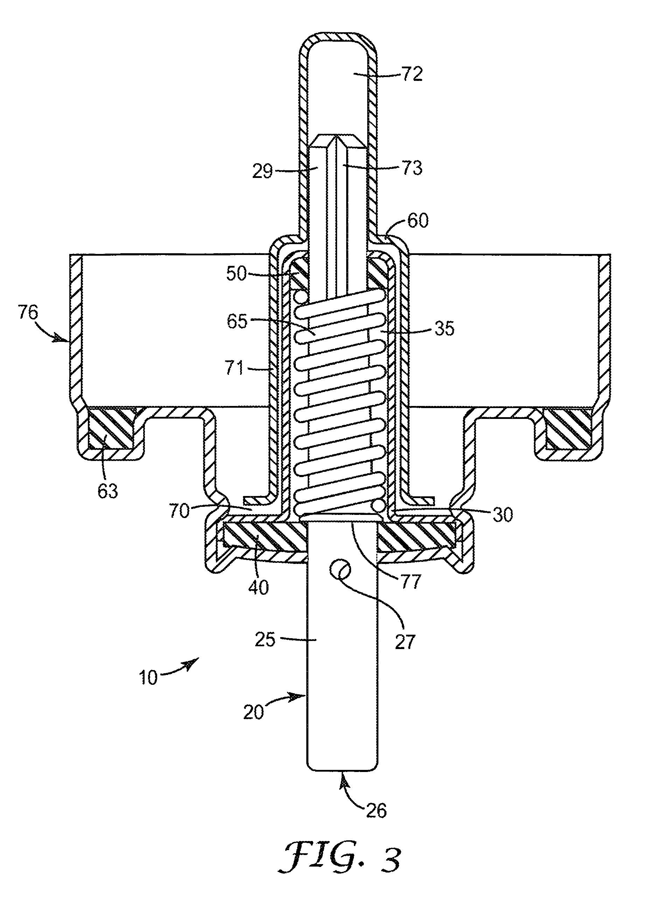

Figure 3 provides a partial cross-sectional view of another exemplary metered dose dispensing valve (10). Similar to the valves shown in Figures 1 and 2, in use, the valve is crimped onto an aerosol container (not shown) via a ferrule (76), and a gasket seal (63) is provided to ensure a gas tight seal. Referring to Figure 3, the valve (10) comprises a valve stem (20) that generally defines a longitudinal axis and a valve body (30), where the valve stem extends through a central aperture of the valve body. A lower stem portion (25) of the valve stem extends outwardly and is in slidable, sealing engagement with a diaphragm seal (40), while an upper stem portion (29) of the valve stem extends inwardly and is in slidable, sealing engagement with a metering gasket (50). A (non-transistory) metering chamber (35) is defined within the valve body (30) between the diaphragm seal (40) and metering gasket (50). A compression spring (65) is positioned within the valve body with one end abutting the metering gasket (50) and the other end abutting a flange (77) on the valve stem near the diaphragm seal. As will be appreciated by the skilled reader, upon movement of the valve stem inwardly, a groove (73) in the upper stem portion (29) will pass beyond the metering gasket (50) so that a complete seal is formed between the upper stem portion of the valve stem and the metering gasket, thereby sealing off the metering chamber. Upon further movement of the valve stem inwardly, an opening (27) into a discharge passageway (26, not visible since the valve stem is not shown in cross section) of the valve stem passes the diaphragm seal into the metering chamber and the contents of the metering chamber pass through the discharge passageway of the valve stem, exiting the stem outlet. Referring to Figure 3, the valve may comprise a second valve body (60) defining a bottle emptier. When such a secondary valve body is provided, aerosol formulation in the aerosol container (not shown) will pass through a gap (70) between the first and second valve bodies (30 and 60) (the gap is near the diaphragm seal), through an annular gap (71) into a pre-metering region (72) and then through the groove (73) into the metering chamber (35). The valve stem (20), primary valve body (30) and/or secondary valve body (60) of such valves are favorably manufactured via CPIM.

The present invention also includes methods of manufacturing a ceramic component of a medicinal pressurized metered dose dispenser (e.g. a ceramic container or a ceramic valve component of a metered dose dispensing valve for use in a medicinal pressurized metered dose dispenser (such as a pMDI)). Such methods typically comprise the steps:
injecting into a mold for the component a feedstock of ceramic particles and/or
ceramic precursor particles in a binder to provide a green part;
removing binder to provide a brown part; and
sintering the brown part to provide a metal part.

Ceramic particles and/or ceramic precursor particles (hereinafter generally referred to as particles) used in the CPIM feedstock are favorably selected such that the manufactured component is made of a ceramic, nitride or a composite thereof. Examples include silicon nitride, aluminum nitride, silicon oxynitride, aluminum oxynitride, boron nitride, and composites thereof.

Components made of a ceramic nitride or composites thereof) have desirable properties (e.g. mechanical and/or thermal properties) for use in a medicinal pressurized metered dose dispenser.

For enhanced structural integrity during filling operations (e.g. cold or pressure filling) and/or use over the lifetime of a medicinal pressurized metered dose dispenser, such as a pMDI, components made of silicon nitride; silicon nitride-containing composites (e.g. SiAlON (i.e. alumina-silicon nitride composite), silicon carbide-SiAlON, and silicon carbide-silicon nitride composites); aluminum nitride; aluminum nitride-containing composites (e.g. alumina-aluminum nitride composites); silicon oxynitride; silicon oxynitride-containing composites (e.g. silicon oxynitride-silicon carbide and silicon oxynitride-zirconium oxide composites); aluminum oxynitride; and aluminum oxynitride-containing composites (e.g. aluminum oxynitride-titanium nitride composites) are particularly favorable.

A small particle size is desirable as it tends to lead to a better (smoother) surface texture (e.g. roughness on a scale of generally less than 3 microns and more favorably less than I micron) on the finished component and very low porosity in the final component. Finer particles also allow for faster de-binding and sintering processes, with consequent financial benefits. Preferably the median particle size is about 30 microns or less, more preferably about 20 microns or less, and even more preferably about 10 microns or less, even more preferably about 5 microns or less, and most preferably about 1 micron or less. The use of particles of about 1 micron or less in size provides especially particularly desirable surface finishes, and close to theoretical final densities, i.e. almost 100% of the corresponding bulk materials' density.

It is desirable that the particles have a distribution of controlled and uniform particle sizes. A narrow size distribution range is generally considered preferable as it helps to reduce the tendency for particle segregation at any stage in the handling and molding process, thereby helping to produce more consistent final parts, with even and consistent shrinkage and better dimensional control. Although a broader range of particle sizes can be used in order to improve particle packing density, it has been found that, if a narrow size range is used, particle loading and sintering conditions can be optimized to increase the density of the final part. Preferably, the particles have a size distribution that is 80% or more (by mass) in the size range of about 30 microns or less, more preferably in the range of about 20 microns or less, even more preferably in the range of about 10 microns or less and most preferably in the range of about 5 microns or less. In terms of the aforesaid ranges, it is favorable that the particles have a size distribution that is 80% or more (by mass) in the size range of about 0.1 microns or more and more favorably in the range of about 0.5 microns or more.

Also in order to achieve high packing density, it may be desirable to control aggregation of particles, for example by treatment of particle surfaces. For example particles may be ball milled with 0.5% oleic acid to favorably reduce any potential for particle aggregation and to facilitate flow-ability of particles.

Desirably particles are spherical and/or approximately spherical in shape. Spherical and/or approximately spherical particles tend to prevent particle alignment issues in the final molded parts, leading to better quality, surface finish and dimensional consistency.

Particles can either be provided in the form of particles which have the desired final ceramic composition or can be provided as a mixture of particles of differing compositions which can be blended to give the desired final ceramic composition after sintering. An alternative approach to the use of ceramic particles is to use ceramic precursor particles (e.g. glass particles or metallic particles) which transform and/or otherwise react to form a ceramic before or after molding (e.g. by crystallization or by oxidation of metal). This transformation can take place as part of the solid-state sintering process. Generally it is preferable to use ceramic particles or mixtures of ceramic particles and ceramic-precursor particles. In regard to the latter approach, mixtures of ceramic particles and metallic particles can also be used to produce components made of metal-doped (typically less than 20% metal by volume) ceramics (such materials are sometimes referred to as cermets and typically include metals, such as nickel, cobalt and molybdenum) when the process is performed under conditions (e.g. non-oxidizing conditions) preventing transformation of the metal (e.g. to a non-metallic inorganic oxide). Generally, however, it is desirable to provide components made of a ceramic (e.g. a monolithic or composite ceramic) substantially free or free of metallic (e.g. nickel, cobalt or molybdenum metal) doping.

Particles for CPIM are commercially available, e.g. from Alcoa Chemie GmbH. Also commercially available ceramic microspheres, e.g. from 3M, may be used.

Particles are blended in a binder to form a feedstock to be used for injection molding.

The principle function of a binder is to (initially) bind the particles (e.g. holding a molded green-part together when the part is removed from the mold) and provide lubrication. A variety of different binders, e.g. binder systems, can be used in CPIM. Typically multicomponent binders are used, including a variety of different components performing different functions. Typical feedstocks comprise 10-50% (by weight) of a binder (e.g. a binder system) with the balance being the ceramic particles and/or ceramic-precursor particles. It is favorable to have the ceramic-particle and/or ceramic-precursor particle loading as high as is practical, in order to minimize part shrinkage and deformation during sintering and densification; preferably at least about 65% by weight particle loading, more preferably at least 80%.

As mentioned above, typically a binder comprises multiple components. In particular it has been found useful to provide a binder comprising a plurality of components having different melting points. The lowest melting point component can thus be removed first, as the part is subsequently heated up to de-binder it, followed by higher melting temperature components. Preferably, a single high melting point component may be left to hold the "brown" part together until the start of the sintering stage. As an alternative to a binder comprising meltable binder components, a binder comprising a plurality of components having different thermal decomposition points may be used.

Typical binders are based on polyolefin thermoplastic materials, such as low molecular weight polyethylene or polypropylene. Other suitable binders include systems based on polyacetal, polystyrene, polyvinylchloride, polyethylene carbonate, polyoxymethylene or polyethylene glycol. Preferably, a binder comprising a polyolefin and/or a polyacetal is used. More preferably, a binder comprising two or more polyolefins, polyacetals, polyolefin waxes and/or polyacetal waxes having different melting points is used.

Alternatively, binders may be suitably water-based. For example, a water-based agar or a water-soluble component based on polysaccharides, cellulose or gelatin can be used as a binder. For example, the water based agars can form a gel network that binds the particles together.

A binder also typically includes a component to act as a lubricant, to facilitate the flow-ability of the feedstock when heated, allowing the feedstock to be injected into the mold. Suitable lubricants include for example waxes (e.g., paraffin wax, microcrystalline wax, Carnauba wax), stearic acid and water-soluble polymers. For wax based lubricant systems, multiple waxes may be used together to make up the lubricant. A combination of two or more polyolefins and/or polyacetals with a wax is one of the most commonly and suitably used binders.

Additional components of a binder system can include resins (e.g. thermoplastic resins), plasticisers and surfactants. Other additives may also be used. These can include for example elasticisers, antioxidants, and organometallic coupling agents. For example, about 1% of stearic acid can be added to act as a surfactant and a mold release agent. The selection of additional components can readily be made by those skilled in the art, based on the size of the desired parts, their required dimensional tolerances and surface finish characteristics, the acceptable cost limits, etc.

The preparation of the feedstock, e.g. addition of components of a binder to ceramic particles and/or ceramic-precursor particles, can be carried out in multiple ways. These include both dry processes (e.g. dry blending, dry milling, or fluidization techniques) and wet processes (e.g. wet milling or slurry mixing). Different individual components might be added by different techniques. For example, spray coating can be used to apply liquid components (e.g. surfactants) to the powder particles, etc. Mixing may take place in an inert atmosphere or under a vacuum where desired, e.g. to avoid undesired oxidation of particles. The most appropriate blending or compounding approaches will depend on the constituents to be mixed to make the chosen feedstock, and such techniques are known to those skilled in the art of CPIM, where the prime objective is to ensure adequate homogeneity of the feedstock. For example for a feedstock including a binder based on thermoplastic polymers (e.g. polyolefins) plus a wax, a preferred approach is to pass blended particles into a heated kneader/mixer system, where the molten wax components are added and kneaded in, followed similarly by molten thermoplastic polymers. The feedstock thus mixed is then cooled and mechanically granulated into desirably uniform granules/pellets of a few millimeters across.

Feedstocks for CPIM are also commercially available, for example from Inmatec Technologies GmbH or BASF.

Once the feedstock is prepared, the procedure of injection into the mold in CPIM is similar to the injection of feedstock in plastic injection molding, and CPIM allows the prepared feedstock to be readily injection molded into very many different configurations, including small and intricate features. The molding presses used (e.g. with heated screw-feed injection systems) and the molds used (e.g. hot runner multi-cavity molds) are also similar to those used for standard plastic injection molding. The same rules of part and mold design also apply, with respect to such considerations as wall thicknesses and their consistency, draft angles, tooling parting lines, gas vents, injection gates, ejector pins, undercuts, shut-outs, etc. The principal difference is that CPIM molds need to have oversize dimensions to allow for the shrinkage that occurs during sintering, so that the final part is to the required dimensions. Shrinkage rates are predictable and are well understood by those skilled in the art, so final tolerances can be reasonably well controlled.

Temperatures used during injection of the feedstock into the mold arc those appropriate to allow for adequate melt flow of the particular binder system, typically in the range from about 180 to about 300°C.

Typically after injection, the part is held briefly in the mold until it has cooled enough to eject. Cooling channels and coolant circulation in the mold may be used to accelerate this process, in order to reduce cycle times. Ejection from the mold is entirely analogous to that employed in standard plastic injection molding. Parts may be released entirely by gravity, but preferably suitably placed ejector pins are used to strip parts from the molding tool cavity.

If desired a green part could be machined at this stage, if any machining operations were desired and/or needed. This is called "soft machining". Generally parts for medicinal pressurized metered dose dispensers (e.g. containers or valve components (e.g. valve stems and/or valve bodies)) do not require any such operations.

As mentioned above there are various different binders, and differing de-binding processes are typically applied for differing binders. The principle methods for de-binding include thermal, solvent and catalytic de-binding processes. The prepared green part may or may not need support at this stage.

Thermal de-binding processes can be conducted in either a static furnace with a temperature/time profile control system, or in a continuous conveyor belt furnace. Often, a de-binding process is arranged to lead directly into the sintering process. Because of the long overall cycle times typically involved (often many hours) in de-binding and sintering, static furnaces can be more cost-effective for most applications.

Specific details of a particular thermal de-binding process depend on the nature and composition of the binder used. For example, for a binder comprising one or more waxes and organic polymer components, such as polyolefins or polyacetals, the following general de-binding process can be used. Typically the wax or waxes are first removed by gradually heating the green part to a temperature between about 80°C and about 120°C. Generally a heating rate is applied which docs not exceeding 300°C per hour, preferably not exceeding 100°C per hour. Temperature holds may be employed at temperatures at which different wax components of the binder will melt and run out of the green part. Green parts may be placed on a bed of alumina powder to facilitate removal of wax components, e.g. to pull the molten wax(es) out by capillary action. Removal of wax(es) leaves very fine channels through the material of the part, through which other components of the binder (e.g. polymeric components) can subsequently pass upon their removal. Once sufficient time has been allowed for the wax component(s) to be melted out of the part, the temperature can be raised further by controlled heating to temperatures at which the organic polymer components are volatilized. Typically, this involves ramping the temperature up to between about 300°C and about 400°C, although depending on the particular components of the binder, temperatures of 600°C or more can be used, even up to 900°C or more. The heating rate typically does not exceed 100°C per hour. Similar to the process used in the removal of wax components, multiple holds at different temperatures may be employed, holding at temperatures corresponding to the volatilization or thermal de-polymerization (pyrolysis) temperatures of the different constituents of the binder.

A variety of different atmospheres may be used during thermal de-binding processes. Air, vacuum (e.g. < 10 mbar), inert gas (e.g. argon), or a reducing atmosphere (e.g. dry hydrogen or Naton (10% hydrogen, 90% nitrogen)) may be used.

As is well known to those skilled in the art, thermal cycles are typically designed to avoid distortion or damage to the initial green part and the resulting brown part after de-binding. For example, in general, rapid heating to elevated temperatures is avoided, so that volatile components of the binder do not out-gas at rates greater than the gas can leak or diffuse out. If a water-based binder system is used, often a pre-drying step (e.g. a slow heating to 110°C) is employed. As another example, typically the rate of generation of liquid melt during de-binding is kept low because if too much of the binder, for example wax component(s), is melted at the same time, the resultant liquid flow can lead to slumping of the parts. Depending on component size and/or binder composition, particular cycles of gradual and staged temperature rises are chosen such as to avoid excess thermal stresses or softening or melting of the parts, while allowing for a reasonable de-binding rate.

Solvent de-binding processes may be used alone or in combination with other methods. For example, much of the binder may be washed out or extracted with a solvent, leaving behind a thermosetting component that can be hardened by exposure to ultraviolet radiation. Alternatively, most of the binder may be flushed out with a solvent (or a vapor), leaving residual binder and, if applicable, residual solvent to be removed by thermal methods. Mineral spirits, hexane and water are examples of solvents used to remove binder components. Similar to thermal de-binding processes the skilled person understands that for solvent de-binding the particular process used is chosen so as to avoid risk of part distortion while allowing for a reasonable de-binding rate.

Catalytic de-binding processes typically involve the addition of a catalyst into the binder, or alternatively a catalyst can be circulated through a de-binding oven in a purge gas flow. In such processes the catalyst facilitates the break up of molecules, e.g. polymeric molecules, of binder components into smaller molecules having relatively high vapor pressures which can then be removed at relatively low temperatures. For example, acid catalysts can be used to break up polyacetal binder components into formaldehyde. Catalytic de-binding processes are desirable in that temperatures as low as 100-150°C can be used to achieve de-binding, thus minimizing any potential of thermal distortion of a part.

Upon de-binding of a green part, a brown part is provided. Such brown parts typically have little strength and are very fragile until sintering together of the particles takes place. Typically, brown parts are primarily held together by a small amount of residual binder that is finally removed during the subsequent sintering process, e.g. by decomposition at or around the higher temperatures used for sintering.

During sintering, brown parts are heated to temperatures high enough to cause the particles to bond together in the shape of the desired part. This bonding is generally a solid state fusion process. For particles comprising ceramic precursor materials, bonding generally occurs as a result of their transformation to the corresponding ceramic, which then fuse together in the solid state.

Typical sintering temperatures are 1200 - 1800°C. Suitable selection of sintering heating rates and holding times are well known to those skilled in the art. For example heating is typically performed at rates that prevent excessive distortion of the part, while sintering times are typically long enough to allow for any required solid state diffusion to occur. Static or continuous sintering furnaces can be used; the latter are common. Sintering may be performed in an inert, reducing or an oxidizing atmosphere.

By the end of the sintering process, part shrinkage will be complete and can often reach 30% or more. Typically shrinkage is on the order of about 20%. Surprisingly for a particular feedstock and a desired valve component form, shrinkage can be predicted and/or controlled so well that ceramic valve components (in particular valve stems and/or valve bodies) for medicinal metering dose dispensing valves can be produced that meet the stringent dimensional tolerances of these very demanding applications.

Sintering leads to the elimination of any residual components of the binder and to densification and strengthening of the part. Final densities of the sintered part are desirably at least 95% or more, more desirably 98% or more, or even more desirably at least 99% or more, of the bulk density of the ceramic. What little porosity remains does not result in any propellant leakage or passage through CPIM-manufactured components during their routine use in a pressurized medicinal dispensing device (e.g. a pMDI). Moreover, components are favorably non-permeable to liquids and gases under the conditions of routine use in a pressurized medicinal dispensing device (e.g. a pMDI).

After sintering, post-CPIM operations may be carried out on the resulting part as desired and/or needed. For example surface polishing may be performed. However it has been surprisingly found that typically no post-CPIM operations, such as polishing or machining, are needed. Moreover it has been advantageously found that the resulting ceramic part obtained after sintering can be used directly as a component of a pressurized medicinal dispensing device (e.g. a pMDI) without any further processing. Thus CPIM processes as described herein allow for the ready and inexpensive manufacture of ceramic components for medicinal pressurized metered dose dispensers (e.g. pMDls), in particular containers and valve components for medicinal metered dose dispensing valves (e.g. valve stems and/or valve bodies), without the need for expensive machining, polishing and/or other post surface-conditioning operations, such as coating.

## Claims

1. A method of manufacturing a ceramic component of a medicinal pressurized metered dose dispenser, said method comprising the steps of :
a) providing a mold for the component,
b) injecting into the mold a feedstock of ceramic particles and/or ceramic-precursor particles in a binder to provide a green part;
c) removing binder to provide a brown part; and
d) sintering the brown part;
**characterized in that** the ceramic particles and/or ceramic precursor particles are selected such that the manufactured component is made of a ceramic nitride or a ceramic nitride-containing composite.

2. A method according to claim 1 in which the density of the manufactured component has a density of at least 95% of the bulk density of the ceramic.

3. A method according to claim 1 or 2 in which the ceramic particles and/or ceramic precursor particles are selected such that the manufactured component comprises silicon nitride, aluminum nitride, silicon oxynitride, aluminum oxynitride, boron nitride or composites thereof.

4. A method according to claim 3 in which the ceramic particles and/or ceramic precursor particles are selected such that the manufactured component comprises silicon nitride-containing composite, aluminum nitride-containing composite, silicon oxynitride-containing composite, or aluminum oxynitride-containing composite.

5. A method according to claim 4 in which the ceramic particles and/or ceramic precursor particles are selected such that the manufactured component is made of a composite comprising SiAlON, silicon carbide-SiAlON, silicon carbide-silicon nitride, alumina-aluminum nitride, silicon oxynitride-silicon carbide, silicon oxynitride-zirconium oxide, or aluminum oxynitride-titanium nitride.

6. A method according to any one of claims 1 to 5 in which the median particle size of the ceramic particles and/or ceramic precursor particles of the feedstock is about 30 microns or less; and/or wherein the particle size distribution of the ceramic particles and/or ceramic precursor particles of the feedstock is 80% or more by mass in the size range of about 30 microns or less; and/or wherein the particle size distribution of the metal particles and/metal-containing precursor particles of the feedstock is 80% or more by mass in the size range of about 0.1 microns or more; and/or wherein the ceramic particles and/or ceramic precursor particles of the feedstock are spherical and/or substantially spherical; and/or wherein the feedstock comprises 65% or more by mass of ceramic particles and/or ceramic precursor particles.

7. A component of a medicinal pressurized metered dose dispenser, the component being made of a ceramic nitride or a ceramic nitride-containing composite, and wherein the component is a valve component for use in a medicinal pressurized metered dose dispenser.

8. A component of a medicinal pressurized metered dose dispenser as claimed in claim 7, the component being made of a ceramic selected from the group consisting of silicon nitride, silicon nitride-containing composite, aluminum nitride, aluminum nitride-containing composite, silicon oxynitride, silicon oxynitride-containing composite, aluminum oxynitride, and aluminum oxynitride-containing composite.

9. A component according to claim 8 in which the component is made of a composite comprising SiAlON, silicon carbide-SiAlON, silicon carbide-silicon nitride, alumina-aluminum nitride, silicon oxynitride-silicon carbide, silicon oxynitride-zirconium oxide, or aluminum oxynitride-titanium nitride.

10. A component according to any one of claims 7 to 9 in which the component has a density of at least 95% of the bulk density of the ceramic.

11. A component according to any one of claims 7 to 10 in which the component is a component that in its use in the pressurized metered dose dispenser comes into contact with pharmaceutical aerosol formulation and/or pressurized, liquefied propellant.

12. A medicinal pressurized metered dose dispenser comprising a component obtained according to a method of any one of claims 1 to 6 or a component according to claims 7 to 11.

## Patentansprüche

1. Verfahren zum Herstellen einer Keramikkomponente eines druckbeaufschlagten Medikamentendosierspenders, wobei das Verfahren die folgenden Schritte umfasst:
a) Bereitstellen einer Form für die Komponente,
b) Einspritzen eines Rohstoffs aus Keramikteilchen und/oder Keramikvorläuferteilchen in einem Bindemittel in die Form, um einen Grünkörper bereitzustellen;
c) Entfernen von Bindemittel, um einen Braunkörper bereitzustellen; und
d) Sintern des Braunkörpers;
**dadurch gekennzeichnet, dass** die Keramikteilchen und/oder Keramikvorläuferteilchen so ausgewählt sind, dass die gefertigte Komponente aus einem Keramiknitrid oder einem Keramiknitrid enthaltenden Verbundstoff hergestellt ist.

2. Verfahren nach Anspruch 1, wobei die Dichte der gefertigten Komponente eine Dichte von mindestens 95 % der Rohdichte der Keramik aufweist.

3. Verfahren nach Anspruch 1 oder 2, wobei die Keramikteilchen und/oder Keramikvorläuferteilchen so ausgewählt sind, dass die gefertigte Komponente Siliciumnitrid, Aluminiumnitrid, Siliciumoxynitrid, Aluminiumoxynitrid, Bornitrid oder Verbundstoffe davon umfasst.

4. Verfahren nach Anspruch 3, wobei die Keramikteilchen und/oder Keramikvorläuferteilchen so ausgewählt sind, dass die gefertigte Komponente einen Siliciumnitrid enthaltenden Verbundstoff, einen Aluminiumnitrid enthaltenden Verbundstoff, einen Siliciumoxynitrid enthaltenden Verbundstoff oder einen Aluminiumoxynitrid enthaltenden Verbundstoff umfasst.

5. Verfahren nach Anspruch 4, wobei die Keramikteilchen und/oder Keramikvorläuferteilchen so ausgewählt sind, dass die gefertigte Komponente aus einem Verbundstoff hergestellt ist, der SiAlON, Siliciumcarbid-SiAlON, Siliciumcarbid-Siliciumnitrid, Tonerde-Aluminiumnitrid, Siliciumoxynitrid-Siliciumcarbid, Siliciumoxynitrid-Zirconiumoxid oder Aluminiumoxynitrid-Titannitrid umfasst.

6. Verfahren nach einem der Ansprüche 1 bis 5, wobei die mittlere Teilchengröße der Keramikteilchen und/oder der Keramikvorläuferteilchen des Rohstoffs etwa 30 Mikrometer oder weniger beträgt; und/oder wobei die Teilchengrößenverteilung der Keramikteilchen und/oder der Keramikvorläuferteilchen des Rohstoffs 80 % oder mehr Massenanteil im Größenbereich von etwa 30 Mikrometer oder weniger beträgt; und/oder wobei die Teilchengrößenverteilung der Metallteilchen und/oder der Metall enthaltenden Vorläuferteilchen des Rohstoffs 80 % oder mehr Massenanteil im Größenbereich von etwa 0,1 Mikrometern oder mehr beträgt; und/oder wobei die Keramikteilchen und/oder die Keramikvorläuferteilchen des Rohstoffs kugelförmig und/ oder im Wesentlichen kugelförmig sind; und/oder wobei der Rohstoff 65 % oder mehr Massenanteil an Keramikteilchen und/oder Keramikvorläuferteilchen umfasst.

7. Komponente eines druckbeaufschlagten Medikamentendosierspenders, wobei die Komponente aus Keramiknitrid oder aus einem Keramiknitrid enthaltenden Verbundstoff hergestellt ist und wobei es sich bei der Komponente um eine Ventilkomponente zur Verwendung in einem druckbeaufschlagten Medikamentendosierspender handelt.

8. Komponente eines druckbeaufschlagten Medikamentendosierspenders nach Anspruch 7, wobei die Komponente aus einer Keramik hergestellt ist, die aus der Gruppe bestehend aus Siliciumnitrid, einem Siliciumnitrid enthaltenden Verbundstoff, Aluminiumnitrid, einem Aluminiumnitrid enthaltenden Verbundstoff, Siliciumoxynitrid, einem Siliciumoxynitrid enthaltenden Verbundstoff, Aluminiumoxynitrid und einem Aluminiumoxynitrid enthaltenden Verbundstoff ausgewählt ist.

9. Komponente nach Anspruch 8, wobei die Komponente aus einem Verbundstoff hergestellt ist, der SiAlON, Siliciumcarbid-SiAlON, Siliciumcarbid-Siliciumnitrid, Tonerde-Aluminiumnitrid, Siliciumoxynitrid-Siliciumcarbid, Silicium-oxynitrid-Zirconiumoxid oder Aluminiumoxynitrid-Titannitrid umfasst.

10. Komponente nach einem der Ansprüche 7 bis 9, wobei die Komponente eine Dichte von mindestens 95 % der Rohdichte der Keramik aufweist.

11. Komponente nach einem der Ansprüche 7 bis 10, wobei es sich bei der Komponente um eine Komponente handelt, die bei ihrer Verwendung in dem druckbeaufschlagten Medikamentendosierspender mit pharmazeutischen Aerosolformulierungen und/oder druckbeaufschlagtem, verflüssigtem Treibmittel in Kontakt kommt.

12. Druckbeaufschlagter Medikamentendosierspender, der eine Komponente, die gemäß einem Verfahren nach einem der Ansprüche 1 bis 6 erhalten wurde, oder eine Komponente nach einem der Ansprüche 7 bis 11 umfasst.

## Revendications

1. Procédé de fabrication d'un composant céramique d'un distributeur-doseur médicinal sous pression, ledit procédé comprenant les étapes consistant à :
a) fournir un moule pour le composant,
b) injecter dans le moule un produit de départ de particules céramiques et/ou de particules de précurseur de céramique dans un liant pour fournir une pièce verte ;
c) éliminer le liant pour fournir une pièce brune ; et
d) fritter la pièce brune ;
**caractérisé en ce que** les particules céramiques et/ou les particules de précurseur de céramique sont choisies de telle sorte que le composant fabriqué est constitué d'un nitrure de céramique ou d'un composite contenant du nitrure de céramique.

2. Procédé selon la revendication 1, dans lequel la masse volumique du composant fabriqué a une masse volumique d'au moins 95 % de la masse volumique en vrac de la céramique.

3. Procédé selon la revendication 1 ou 2, dans lequel les particules céramiques et/ou les particules de précurseur de céramique sont choisies de telle sorte que le composant fabriqué comprend du nitrure de silicium, du nitrure d'aluminium, de l'oxynitrure de silicium, de l'oxynitrure d'aluminium, du nitrure de bore ou des composites de ceux-ci.

4. Procédé selon la revendication 3, dans lequel les particules céramiques et/ou les particules de précurseur de céramique sont choisies de telle sorte que le composant fabriqué comprend un composite contenant du nitrure de silicium, un composite contenant du nitrure d'aluminium, un composite contenant de l'oxynitrure de silicium, ou un composite contenant de l'oxynitrure d'aluminium.

5. Procédé selon la revendication 4, dans lequel les particules céramiques et/ou les particules de précurseur de céramique sont choisies de telle sorte que le composant fabriqué est constitué d'un composite comprenant du SiAlON, du carbure de silicium-SiAlON, du carbure de silicium-nitrure de silicium, de l'alumine-nitrure d'aluminium, de l'oxynitrure de silicium-carbure de silicium, de l'oxynitrure de silicium-oxyde de zirconium, ou de l'oxynitrure d'aluminium-nitrure de titane.

6. Procédé selon l'une quelconque des revendications 1 à 5, dans lequel la taille moyenne de particules des particules céramiques et/ou de particules de précurseur de céramique du produit de départ est d'environ 30 micromètres ou moins ; et/ou dans lequel la distribution granulométrique des particules céramiques et/ou des particules de précurseur de céramique du produit de départ est de 80 % ou plus en masse dans la plage de taille d'environ 30 micromètres ou moins ; et/ou dans lequel la distribution granulométrique des particules de métal et/des particules de précurseur contenant du métal du produit de départ est de 80 % ou plus en masse dans la plage de taille d'environ 0,1 micromètre ou plus ; et/ou dans lequel les particules céramiques et/ou les particules de précurseur de céramique du produit de départ sont sphériques et/ou sensiblement sphériques ; et/ou dans lequel le produit de départ comprend 65 % ou plus en masse de particules céramiques et/ou de particules de précurseur de céramique.

7. Composant d'un distributeur-doseur médicinal sous pression, le composant étant constitué d'un nitrure de céramique ou d'un composite contenant du nitrure de céramique, et où le composant est un composant de soupape destiné à être utilisé dans un distributeur-doseur médicinal sous pression.

8. Composant d'un distributeur-doseur médicinal sous pression selon la revendication 7, le composant étant constitué d'une céramique choisie dans le groupe constitué de nitrure de silicium, composite contenant du nitrure de silicium, nitrure d'aluminium, composite contenant du nitrure d'aluminium, oxynitrure de silicium, composite contenant de l'oxynitrure de silicium, oxynitrure d'aluminium, et composite contenant de l'oxynitrure d'aluminium.

9. Composant selon la revendication 8, où le composant est constitué d'un composite comprenant du SiAlON, du carbure de silicium-SiAlON, du carbure de silicium-nitrure de silicium, de l'alumine-nitrure d'aluminium, de l'oxynitrure de silicium-carbure de silicium, de l'oxynitrure de silicium-oxyde de zirconium, ou de l'oxynitrure d'aluminium-nitrure de titane.

10. Composant selon l'une quelconque des revendications 7 à 9, où le composant a une masse volumique d'au moins 95 % de la masse volumique en vrac de la céramique.

11. Composant selon l'une quelconque des revendications 7 à 10, où le composant est un composant qui lors de son utilisation dans le distributeur-doseur sous pression vient en contact avec une formulation d'aérosol pharmaceutique et/ou un propulseur liquéfié sous pression.

12. Distributeur-doseur médicinal sous pression comprenant un composant obtenu selon un procédé selon l'une quelconque des revendications 1 à 6 ou un composant selon les revendications 7 à 11.
